Europäisches Patentamt

European Patent Office

Office européen des brevets

(11) Publication number: 0 301 699
A2

# EUROPEAN PATENT APPLICATION

(21) Application number: 88305732.5

(22) Date of filing: 23.06.88

(51) Int. Cl.4: G01N 21/51 , G01N 21/25 , G01N 33/04 , G01N 33/15

(30) Priority: 23.06.87 US 66000

(43) Date of publication of application:
01.02.89 Bulletin 89/05

(84) Designated Contracting States:
AT BE CH DE ES FR GB GR IT LI LU NL SE

(71) Applicant: Utah State University Foundation
Utah State University
Logan Utah 84322(US)

(72) Inventor: Richardson, Gary H.
1550 East 1220 North
Logan, Utah 84322(US)

(74) Representative: Harrison, Michael Robert et al
Urquhart-Dykes & Lord 91 Wimpole Street
London W1M 8AH(GB)

(54) Method and apparatus for determining the bioactivity of biological samples.

(57) Methods and apparatus for determining the bioactivity of liquid biological mixtures. The method of the present invention involves obtaining a suitable quantity of liquid biological mixture. Once the sample is obtained, visual spectrum light is directed into the liquid biological mixture and reflectance of the light from the mixture is detected and quantified.

The apparatus of the present invention incorporates a microcolorimeter with high energy source as a reflectance detector. The microcolorimeter is able to measure energy in a smaller area, such as 3 to 8 millimeters in diameter, and provide estimates of bioactivity in less than 30 minutes to 32 hours, as opposed to the 24 hour to 9 days which is typical of traditional microbiological methods.

The method of the present invention could involve continuous measurements by recycling plates past the detector at regular intervals until the end point is detected. Thus, the detector could be fitted with an appropriate incubator environment which would have controlled robotic devices to receive, identify, and position plates for storage, reading and for discard. Data is collected and compared by computer. The computer calculates the desired information on bioactivity.

By providing a detector that produces "tristimulus" color values it is possible to use traditional dyes used in microbiological research and testing. Even abandoned dyes could be restored to useful measurement including litmus, methylene blue, and resazurin.

## METHOD AND APPARATUS FOR DETERMINING THE BIOACTIVITY OF BIOLOGICAL SAMPLES

### The Field of the Invention

The present invention relates to improved methods for quickly and efficiently determining characteristics of a specimen, including bioactivity using reflected visual spectrum light as a detection means. More specifically, the present invention relates to methods and apparatus for determining the bioactivity of dairy products, pharmaceutical formulations, environmental samples and related substances.

### The Background of the Invention

Complex procedures have been developed in the food processing industry for assuring food quality. This is particularly true with regard to dairy products. Quality control begins with the raw ingredients and continues through each stage of the processing, packaging, storage, and shipment of the products. Any defect which may occur at any one of these stages may result in a degraded and adulterated product. This is particularly true with some dairy products which are easily spoiled by exposure to contamination or excessive heat and light.

In order to ensure that high quality products reach the consumer, elaborate quality assurance and quality control programs are needed and have been established. Incoming basic ingredients are closely checked against the processor specifications. Formulation standards are established for each product. Process times, temperatures, sequences, and other variables are documented for a multitude of operations in the processing of food products, particularly dairy products.

Generally, few sophisticated analytical methodologies are used to routinely assure high quality products. These may include near infrared spectrophotometry, and other spectrophotometric methods. In addition, as will be discussed below, numerous microbiological methods are used. These methods are used to measure various quality factors in new ingredients, in process products, and in the finished packaged goods.

With regard to dairy products, "standard methods for examination of dairy products" (SMEDP) have been compiled and promulgated with extensive use in the trade. These methods provide procedures to determine various characteristics of dairy products, as will be discussed in additional detail below. Standardized methods for measuring the bacteriological content of milk, for example, have been in existence since about 1905 when the American Public Health Association ("APHA") established a committee to standardize such methods. SMEDP are used to assure that the consumer receives only high quality dairy food products.

Similar analysis problems have been encountered in related fields. In producing medical and pharmaceutical products quality control, including specifically limiting the number of microorganisms present, is a major concern. Extensive steps must be taken in order to assure a pure product. Likewise, determination of contamination relies on the same general techniques as does quality control. Thus, analysis of the quality of a chosen environment is similar in many respects to the quality control problems mentioned above.

It will be appreciated that the bacterial content is one of the most important measurements in product quality control in the industries mentioned above. Various other factors are, however, also measured in order to assure that high quality medical, pharmaceutical and dairy products are provided to the consumer.

One area of biological testing of dairy food products, pharmaceuticals and related types of products involves the estimation of bacteria, yeasts and molds, and other such microorganisms, within the material. One widely used method is known as the "Standard Plate Count" (SPC) and involves culturing a diluted sample on an agar medium. The plates containing the medium and the sample are then generally incubated at 32°C for about 48 hours. Once the samples have been incubated over this relatively long period, the colonies of microorganisms which grow on the medium are counted manually. As an alternative, some laboratories now use automated colony counters. In any event, the standard plate count method is extremely time consuming, costly, and tedious. At the same time, the method is not particularly useful in that the bacterial formation numbers do not always correlate with the desired parameter being estimated.

Regulatory agencies, such as the Food and Drug Administration, however, often require use of SPC even in view of the limitations encountered using this method.

An additional method of determining the bacterial concentration of milk or other biologically active materials is by the "electrical impedance method." The bacteria, yeast, or mold concentration in a liquid sample is determined by measuring impedance changes associated with the metabolic activity of the reproducing microflora present in each sample when the sample is inoculated onto an agar surface. The microbial activity results in the conversion of nonelectrolytes (such as lactose) to ionic compounds (such as pyruvic acid and lactic acid). Less conductive proteins can be hydrolyzed to more conductive amino acids and fats can be converted to free fatty acids, also more conductive.

Impedance is defined as a resistance to the flow of an alternating electrical current through a medium. Impedance of a sample remains relatively constant until the number of microorganisms present in the incubated sample reaches a threshold of from approximately $10^6$ to $10^7$ organisms per ml. When this threshold is achieved, the rate of impedance change due to changes in the ionic constituency of the medium is measurably increased. Thus, by measuring impedance and the period of time until impedance rapidly changes, an estimate is provided concerning bioactivity.

A limitation on the impedance method is that a single signal is achieved using this method and it is subject to stray electrical interferences. The test is very sensitive to slight temperature variations. There is no visual backup method for assuring that the data has] is accurate and, as a result, false end points may often be encountered.

Electrical conductivity of a sample (such as a milk sample) may also be important in determining other characteristics of the sample. For example, the general determination on mastitic of "abnormal milk" can be made through conductivity measurements. Abnormal milk often occurs when mammary tissues cannot maintain lactose synthesis, resulting in a lack of lactose in the milk. As a result, the nonconductive lactose is replaced in the milk by conductive salts. These salts diffuse through the tissue membranes from the blood serum of the producing animal in order to maintain normal osmolality. This effects a major change in electrical conductivity of the milk that correlates to changes in lactose. These changes can be measured quite readily and give an indication of the existence of abnormal milk. For the purpose of ease of reference, abnormal milk and other similar types or tests discussed herein will be considered to be tests of bioactivity.

Impedance methods allow only about 250 samples per day to be evaluated on a single module instrument. Present techniques require solid media for performance impedance testing. This technique is, therefore, very labor intensive and does not lend itself to automation.

An additional group of tests relate to the coliform group of bacteria. This group of bacteria comprises all aerobic and anaerobic gram negative, nonspore-forming bacteria which are able to ferment lactose with production of acid and gas at 32°C within 24 hours. One of the major sources of these organisms is the intestinal tract of warm-blooded animals, such as dairy cows. These organisms fall generally within the following three genera: escherichia, enterobacter, and klebsiella. A few other types of bacteria may also fall within the general classification of coliform bacteria.

The presence of coliform bacteria is measured in food and dairy product quality control tests because their presence is suggestive of unsanitary conditions or practices during the production, process, or storage of the products. Specifically, post pasteurization contamination may be indicated by this group of organisms. The test for coliforms is not intended specifically to identify any particular bacteria but rather to measure the quality of the general practices used to minimize contamination.

Coliform bacteria are usually detected using plate counting or fermentation techniques. These techniques are similar to the SPC technique described above. Samples are placed onto an agar medium or are added to a selective liquid medium (such as brilliant green lactose bile broth). Plate counting is usually found to be effective when there are more than five colonies per milliliter.

Multiple tube fermentation with liquid medium may be used when it is desired to estimate lower concentrations of bacteria. These techniques suffer from the same limitations as do the other culturing techniques described. These limitations relate primarily to the time and effort needed to obtain reasonably accurate data. Also, the data do not correlate with predicting shelf, for example, as well as some newer instrumental methods, such as impedance methods.

An additional characteristic of milk which is often tested is the somatic cell concentration. Somatic cell concentration in excess of 500,000 per milliliter ("ml") is generally considered to be an indication of abnormal or mastitic milk. Cows with a latent or low grade infection are likely to produce milk having excessive somatic cell concentrations.

One test for somatic cells is the California Mastitis Test (CMT). In that test a reagent which consists of a detergent plus a pH indicator (brom cresol purple) is added to the milk. The extent of the reaction between the reagent added and the DNA in the milk is an indication of the content of somatic cells in the milk.

Other tests have also been devised for testing for somatic cell content. For example, the Wisconsin Mastitis Test measures viscosity after adding a detergent to raw milk. An improvement over the Wisconsin Mastitis Test is the Ruakura rolling ball viscometer. This is an improved method of measuring viscosity which gives an indication as to the somatic cell content. These tests, however, are subject to the limitations set forth below.

Several problems exist in the art with regard to each of the techniques described above. The most serious drawback to existing techniques is the fact that most are all extremely time consuming. Most of the techniques require preincubation of the sample before measurements can begin. As was discussed above, several of the techniques require the culturing of cells on a medium before the measuring technique can take place.

The techniques which require preincubation and culturing of the medium often take from 24 hours to over 9 days before any usable reading or results can be produced. Some impedance techniques which simply require electrical stabilization of the electrodes in order to take an electrical reading may take in excess of 12 hours before samples can be added. As can be appreciated, the time delay is extremely inconvenient and expensive.

Since the techniques are applied to food, medicines, and other perishable products, delaying for several days the marketing of products while the testing occurs can itself result in product being consumed or used before the test is completed. In addition, the number and thoroughness of the test will be limited simply by the time constraints. Only a very limited number of samples can be tested using present known techniques.

One of the further limitations in the existing art is that the testing instruments are not versatile. The testing techniques primarily indicate only one characteristic of the product to which they are applied. A conductivity test, for example, may simply disclose the extent to which conductive salts and other conductive species are contained within a milk sample. Likewise, the culture techniques described above may only give an indication as to the presence or absence of a particular type of bacteria. It has not been possible to use a single testing technique in order to obtain data concerning multiple "bioactivity" characteristics of the food, medical, or environment product.

Many of the techniques described above also require a large volume of sample in order to run the test properly. For example in the standard plate counting technique described above, it is necessary to obtain a relatively large sample and then proceed through several dilutions because of the numbers of bacteria in the sample before the test can occur. It is not practical to simply take a small sample (0.1 ml), for example, and directly use that sample to conduct the test.

An additional limitation of existing techniques is that it is not possible to make fine measurements. Devices that physically measure dead bacterial cells have a lower limit of about log 4 "colony forming units" per ml ("cfu/ml"). When the concentration of bacteria drops significantly below the lower limit it is impossible to obtain accurate data. The technique of the present invention, conversely, is capable of measuring below log 2 cfu/ml.

As mentioned briefly above, one of the primary limitations of existing techniques is that it is only possible to obtain simultaneous data from a very few samples. This is because of the fact that the existing techniques are extremely time consuming and labor intensive. As a result, in the case of the standard plate counting technique it is only practical to obtain data from a very few samples per day. This is clearly limiting in the ability to obtain accurate data and to compare that data with some type of base or standard. Thus, using existing techniques it is only possible to obtain a general indication concerning the bioactivity of the sample.

It is apparent that what is needed in the art are methods and apparatus for determining bioactivity in dairy products, other food products, pharmaceuticals, medicines, environmental samples, and similar substances which overcome the problems described above. Specifically, it would be an advancement in the art to provide methods and apparatus for determining bioactivity which did not require extensive preincubation, culturing, and stabilization of the sample and related equipment before readings could be taken. It would be a related advancement in the art if such methods and apparatus could be provided which allowed readings to be taken in numerous samples more quickly and efficiently than is presently possible.

It would be a further advancement in the art if methods and apparatus could be provided for measuring bioactivity in samples which methods and apparatus were versatile in that several different tests could be performed using essentially the same methods and apparatus. It would also be an advancement in the art if such methods and apparatus could be provided whereby it was simple to obtain end points and base lines for measuring bioactivity. It would be another advancement in the art if readings could be taken on very small undiluted samples and in samples where the bioactivity correlated to product quality.

Such methods and apparatus are disclosed and claimed herein.

## BRIEF SUMMARY AND OBJECTS OF THE INVENTION

The present invention is related to methods and apparatus for determining the bioactivity of biological mixtures or samples, including solutions, emulsions, suspensions, and solids. As used in this application, the term "biological mixture" will refer primarily to mixtures such as dairy products, and other related types of food products, pharmaceuticals, blood, environmental samples, and cosmetics. Bioactivity will refer to the characteristics of the biological mixture including content of various micororganisms, as well as pH, conductivity and other similar characteristics which are influenced by factors such as microorganism content, cell function, and other biological phenomena.

The method of the present invention involves obtaining a suitable quantity of liquid biological mixture. Using the present invention, a comparatively small sample can be used, such as samples which may be placed into Microtiter wells. Once the sample is obtained, visual spectrum electromagnetic energy (visual light) is directed into the liquid biological mixture and reflectance of the energy from the mixture is detected and quantified.

The apparatus of the present invention incorporates a microcolorimeter with a high energy source as a reflectance detector. This microcolorimeter is used in place of transmitance-turbidometric detection systems which are commonly used in microtiter plate well (ELISA) readers, or the impedance detectors which are also sometimes used in measuring biological activity as discussed above. The microcolorimeter is able to measure energy in a small area, such as 3 to 8 millimeters in diameter, and provide estimates of bioactivity in 30 minutes to 32 hours, as opposed to the 24 hours to 9 days which is typical of traditional microbiological methods. The colorimeter is able to make instantaneous estimates of chemical parameters such as NaCl for abnormal milk for example.

Unlike traditional colorimeters, a strobe-like flash occurs at regular intervals to provide sufficient energy for precise and adequate readings of the reflected color. For some purposes it may even be desirable to install reflective surfaces below the plates such that sufficient energy would return for measurement of clear solutions, in addition to turbid or opaque solutions. Thus, for example, it would be possible to measure casein suspensions as they become clear with proteolysis or fat emulsions as they lose turbidity with lipolysis.

The method of the present invention provides the capability to make continuous measurements by recycling plates past the detector and reading each well at regular intervals until the end point is detected. Thus, in one embodiment of the invention, the detector is fitted with an appropriate incubator environment which includes a controlled analyzer robot to rotate plates over the colorimeter. A second sampler robot prepares, pasteurizes, mixes, and preincubates samples. It then inoculates plates and places them on shelves for the analyzer robot. Data is collected and compared by computer. The computer calculates the desired information on bioactivity.

By providing a detector that produces "tristimulus" color values it is possible to use traditional dyes used in microbiological research and testing. Even abandoned dyes could be restored to useful measurement, including litmus, methylene blue, and resazurin dyes.

Tristimulus light is generally defined as light having three different numerical codes. These codes are sometimes designated the X, Y, and Z, or Yxy coordinates under international standards. In the alternative, the coordinates are sometimes designated L* (el star), a* (a-star) or b* (b-star). The L* coordinate light indicates darkness; that is black or white (opaque to clear). The a* coordinate light measures changes from red to green. The b* coordinate light measures color changes from blue to yellow.

The apparatus of the present invention would be useful in clinical and biological research laboratories, dairy and food industry laboratories, in the environmental field and in laboratory applications.

Automated sampling coupled with the incubator/detector easily samples hundreds of these types of samples per hour. The instrument is also usable in the traditional "ELISA" and similar immunological plate reading. As with any similar instrument, pre-enrichment or incubation would be expected where very low levels of microflora are present. For example, to detect 1 colony forming unit ("CFU") per milliliter of coliform organisms, a pre-enrichment of 3 hours, or a total of 14 hours would likely be required for detection. This is, however, a much shorter time than that the 24 hours required using traditional methods.

An example of a series of tests of bioactivity that are automated for the dairy industry using the detector/incubator described above include: abnormal milk; antibiotics; total bacterial count; coliforms; laboratory pasteurized samples; bacterial spores; psychrotrophs; product shelf life stability; sterility; staphylococci; other pathogens; lactic culture activity; bacteriophage tests; yeast in yogurt; cheese contaminants; DNASE activity; lipase; and proteolytic activities. The device can measure other tests like the Delvotest-Multi used for antibiotics. These and other tests are all run using the methods and apparatus of the present invention.

It will be appreciated that the present invention produces results with lower numbers of bacteria much faster than by known methods. Some results are available within one hour (such as abnormal milk, antiobiotics, and culture activity tests). Others take several hours depending on the degree of contamination; however, the results are available much quicker than using known techniques. For example, shelf stability tests take a maximum of about 32 hours, whereas such tests now take up to 9 days.

As a result, it is a general object of the present invention to provide methods and apparatus to measure of biological activity where no extensive pre-incubation, culturing, or electrode stabilization time is required.

Accordingly, it is an object of the present invention to provide methods and apparatus for sampling biological activity whereby the sampling is not time consuming and numerous samples can be analyzed in a short period of time.

It is a further object of the present invention to provide versatile methods and apparatus for measuring biological activity whereby several different biological readings can be taken using the same methods and apparatus.

It is also an object of the present invention to provide methods and apparatus for determining biological activity using reflected visual spectrum energy.

It is a further object of the present invention to provide methods and apparatus for analyzing bioactivity in small samples.

It is a related object of the present invention to provide such methods and apparatus whereby low concentrations of bioactivity can be determined after about log 6 to 7 cfu/ml exist.

It is another object to provide such methods and apparatus where no extensive preincubation, culturing, and stabilization is required for most samples.

It is also an object to provide such methods and apparatus which are capable of quickly reading numerous samples.

These and other objects and advantages of the invention will become apparent upon reading the following detailed description and appended claims, and upon reference to the accompanying drawings.

## BRIEF DESCRIPTION OF THE DRAWINGS

Figure 1 is a cross-sectional plan view of one embodiment of the microcolorimeter of the present invention in combination with a microtiter well.

Figure 2 is an additional embodiment of the colorimeter of the present invention in combination with a microtiter well containing an opaque sample.

Figure 3 is a vertical cross-sectional plan view of the colorimeter of the present invention in combination with a multiple plate stack of microtiter wells illustrating generally robotics combined with the colorimeter.

Figure 4 is a horizontal cross-sectional plan view of the same embodiment of the device illustrated in Figure 3 showing the horizontal placement of the microtiter well plates.

Figure 5 is a bar graph showing the readings obtained by the apparatus of the present invention in the L*, a* and b* tristimulus directions for milk and water (some samples containing BCP dye).

Figure 6 is a graph showing change in the b* value when lactic culture is inoculated into sterile milk.

Figure 7 is a graph showing the changes in the b* value associated with levels of penicillin in sterile BCP milk.

Figure 8 is a graph showing changes in the b* value due to coliform activity.

Figure 9 illustrates changes in a* activity in the same tests as those illustrated in Figure 7, except kanamycin antibiotic and resazurin dye (oxidation/reduction) were used.

Figure 10 is a graph illustrating changes in a* activity in tests similar to those illustrated in Figure 7, except streptomycin antibiotic and triphenyl tetrazolium chloride (TTC) pH dye was used.

Figure 11 is a graph illustrating changes in b* values under circumstances similar to those illustrated in Figure 10 when litmus dye (both pH and o/R indicator) is used.

Figure 12 is a graph illustrating changes in L* values when Escherichia coli ("E coli") bacteria is diluted out, inoculated, and allowed to grow in milk containing BCP dye.

Figure 13 illustrates changes in L* values under similar circumstances to those in Figure 12 using litmus dye.

Figure 14 illustrates changes in L* values under circumstances similar to those shown in Figures 12 and 13 when resazurin dye is used.

Figure 15 illustrates the use of TTC dye in estimating L* values to estimate bacterial numbers.

Figure 16 illustrates changes in b* values when BCP is used in a standard method for antiobiotics detection.

Figure 17 is a plot of somatic cell count against changes in the b* value as a function of milk pH.

Figure 18 shows changes in b* value during incubation of the Delvotest Multi for antibiotics.

Figure 19 shows the change in L* values plotted against changing NaCl values.

Figure 20 shows the changes in b* values against changing concentrations of NaCl.

## DETAILED DESCRIPTION OF THE PREFERRED EMBODIMENTS

## 1. General Description

The present invention provides methods and apparatus for determining the bioactivity of various biological samples. More particularly, the present invention relates to the determination of bioactivity by measuring the reflectance of visual light (visual electromagnetic energy) from a biological medium.

Traditional methods of determining bioactivity included turbidimetric and transmittant light measurements, conductivity measurements, and counting cells in a culture. As discussed above, the present invention has many advantages over any of the prior art methods. For example, it is possible using the present invention to obtain accurate data while using only a very small area of measurement and a very small sample. At the same time, it is possible to continuously measure a sample at frequent intervals.

Accordingly, regularly spaced light pulses may be directed into the biological system and the reflectance may be essentially continuously measured. Thus, fine variations in readings will give very accurate data concerning fine variations in the bioactivity of the sample. At the same time, electrode stabilization times are avoided as are long incubation times as needed in psychrotrophic counts. It is not necessary using the present invention to provide for long periods of stabilization and for long incubations of the sample before accurate measurements can be obtained.

As mentioned above, the present invention utilizes reflected visual spectrum light. This is contrary to the teachings of the existing art which have used transmittance or conductance electrodes. Visual light is often broken down into three different dimensions or values. When these three values are used together the measurement is often referred to as "tristimulus." The three different measurements have different designations in the art. However for the purpose of the present discussion the values in the three different dimensions can be labeled $L^*$, $a^*$, and $b^*$.

As discussed briefly above, $L^*$ is a measurement of how light or dark a sample is (opaque to clear or white to black). The $a^*$ value, similarly, measures changes between red and green and the $b^*$ measures changes from blue to yellow.

When the present invention is combined with various dyes, fine color changes may be detected and may be correlated to changes in the bioactivity of the sample. While employing the present invention it is found that dyes that have traditionally been used in microbiology (particularly in dairy and food), but have lost favor, can again be employed. Some of the traditional dyes which can be used include resazurin, methylene blue, brom cresol purple (BCP), brom thymol blue, TTC, CVT, and litmus. When these dyes change color slightly due to either pH or 0/R changes, or both, the $L^*$, $a^*$, or $b^*$ values will correspondingly change and a measure of the change within the solution will be readily provided.

Using the present invention, a microcolorimeter with a high energy pulse source will be used as a reflectance detector. The device is able to measure changes in a small area such as in a 3 to 8 millimeter diameter zone the typical size of a microtiter well. At the same time, some biological activity will be measured in less than 30 minutes, rather than the 24 hour to 9 day period that is traditionally required for present microbial methods.

Using the present invention a strobe flash occurs at regular intervals to provide sufficient high energy visual light for adequate readings of reflected color. A typical sample will be included in a microtiter well contained within a microtiter plate. Reflective surfaces may be installed below the sample (microtiter plate) in order to allow for accurate measurements in clear solutions. For example, measurements would be possible as suspensions become clear with proteolysis or fat emulsions lose turbidity with lipolysis.

At the same time, it is possible to provide essentially continuous measurement. Flashes of light occur 1 to 2 seconds apart and changes in the sample are rapidly detected. As will be discussed in more detail below with regard to the apparatus, it is also possible to cycle samples past the microcolorimeter as frequently as necessary in order to provide the required data. Thus, continuous data can be obtained if necessary.

It is possible to introduce the data collected into a computer so that the desired biological activity can be rapidly calculated. Thus, bioactivity is quantified and analyzed almost immediately.

As mentioned briefly above, the present invention is used to provide various types of data. This is in direct contrast to existing methods and apparatus which generally provide only one type of reading. The present invention, for example, is adaptable for providing all of the following types of data:

(a) abnormal milk;
(b) antiobiotics;
(c) total bacteria count;
(d) coliforms;
(e) laboratory pasteurized or spore count samples;
(f) psychotrophs;
(g) product shelf life stability;
(h) sterility;
(i) staphylococci and other pathogens;
(j) lactic culture activity;
(k) bacteriophage tests;
(l) yeast in yogurt;

(m) cheese contaminants;

(n) DNAse activity; and

(o) lipidase and proteolytic activities.

Some of the results are available within an hour (such as abnormal milk, antiobiotics, and culture activity tests). Others take several hours depending on the degree of contamination with microflora. Shelf life stability tests, for example, take a maximum of about 32 hours, whereas those tests typically as many as 9 days using existing methods. Only the dyes and media additions would need to be changed and the readout defined for the detector. For example, when brom cresol purple is added to milk samples adjusted from pH 6.4 through 6.8, the range covering normal and abnormal milk, the present invention is able to measure the increase in blue color as the milk becomes abnormal.

Use of narrow band pH dyes would enhance this capability. This result is available with no incubation time. As the samples are incubated and the colors change to shades of yellow, indications of initial bacterial microflora are available in addition to estimates of abnormal milk. The method thus allows the automation of a more precise technique based upon biochemical changes.

## 2. Apparatus of the Present Invention

The apparatus of the present invention generally relates to a microcolorimeter. The microcolorimeter measures changes in the $L^*$, $a^*$, and $b^*$ values of visible light reflected off of a sample. Thus, reflectance is the primary measurement provided by the apparatus of the present invention.

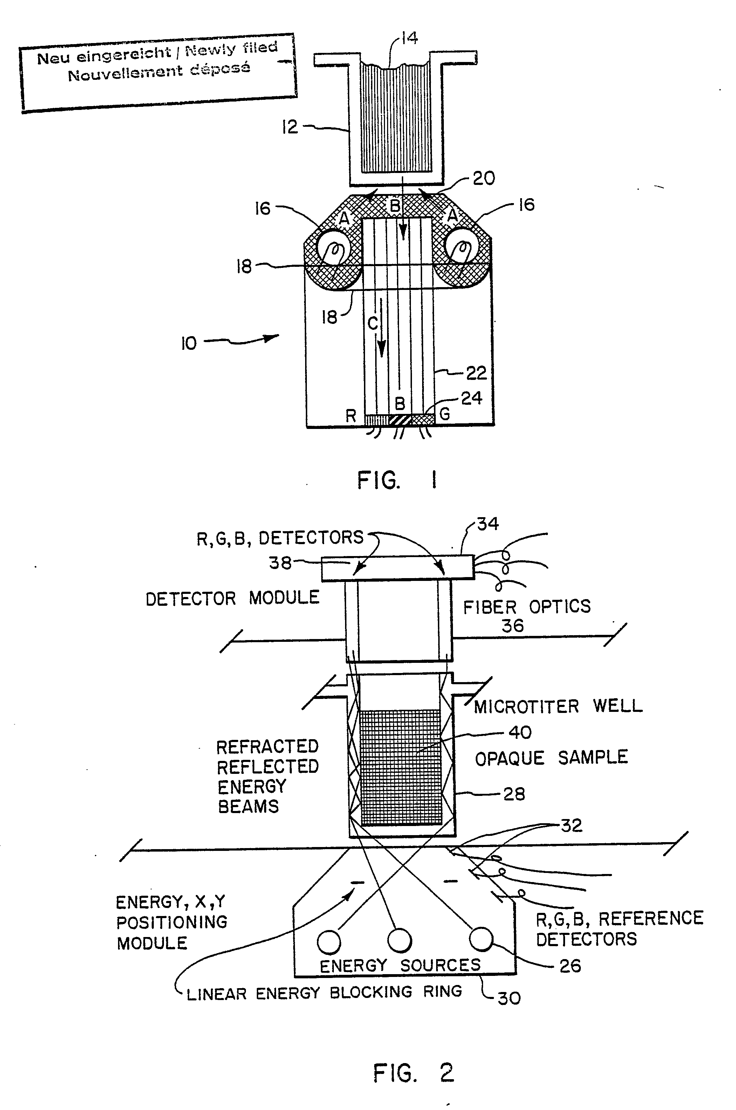

One embodiment of the colorimeter of the present invention is illustrated in Figure 1. The colorimeter of the present invention is generally designated 10 in Figure 1. Also illustrated in Figure 1 is a microtiter well 12. The microtiter well contains a biological mixture, the upper surface of which is designated 14.

The microtiter well may be placed above or below the colorimeter as desired, or, in the alternative, the colorimeter may be placed on any side of a clear sample container. It is only necessary that the device be calibrated so that accurate readings may be obtained when the microtiter well is placed in any particular position with respect to the colorimeter.

The colorimeter will contain one or more energy sources 16. In one embodiment, the energy or light sources may operate in a pulse or strobe-like manner. In the event that they do operate in a pulse or strobe like manner it may be desirable to place reflectors 18 beneath the energy source 16 such that the pulsed light will be reflected in the proper direction and be directed into the microtiter well 12. Thus, the light will follow arrows A illustrated in Figure 1.

A high energy pulse of light, therefore, will be directed out of the optical opening 20 and the light will be directed toward the microtiter well 12. Once the light enters the microtiter well 12, a portion of the light will be reflected. That light will leave the microtiter well 12 traveling along reflected light path B as designated in Figure 1.

Once the reflected light traveling along arrow B reenters the colorimeter 10, it is picked up and carried by fiber optics 22. Fiber optics 22 may continue through the interior of colorimeter 10 and then travel into filter/detectors 24 located at the base of the microcolorimeter 10. The filter/detectors illustrated in Figure 1 include red, blue, and green filtered detectors. A trio of reference detectors would allow traditional double beam operation.

It will be appreciated that microcolorimeter 10 will be placed in combination with known electronic equipment so that the intensities of light along any one of the three tristimulus paths may be monitored at any particular point in time. Such computer hardware and software as necessary is used in order to analyze the pulses detected by the filter/detectors 24. This analysis will include determinations of the variations in $L^*$, $a^*$, and $b^*$ values as appropriate so that extremely fine deferences in color can be detected. A typical computer system usable in the present invention is an IBM compatible microcomputer with adequate storage such as the 30 MB hard disc-containing "Telecat" of Televideo Co.

Figure 2 shows an alternative embodiment of the colorimeter of the present invention. In Figure 2, an energy source 26 is illustrated at the base of the microtiter plate 28. The energy source 26 will be housed within a base housing 30.

The base housing 30 will comprise all of the basic elements of a microcolorimeter, including red, blue, and green detectors 32. The microcolorimeter illustrated in Figure 2, however, has a second component. That second component comprises detectors on the upper surface of the microtiter well 28. The detector is housed within upper housing 34. Upper housing 34 includes fiber optics 36, and red, blue, and green detectors 38.

The embodiment of the device illustrated in Figure 2 includes a detector system to measure the deflected energy bypassing the opaque sample in microtiter well. Thus, additional flexibility in measuring an opaque sample such as opaque sample 40 contained within the microtiter well is provided by the illustrated embodiment.

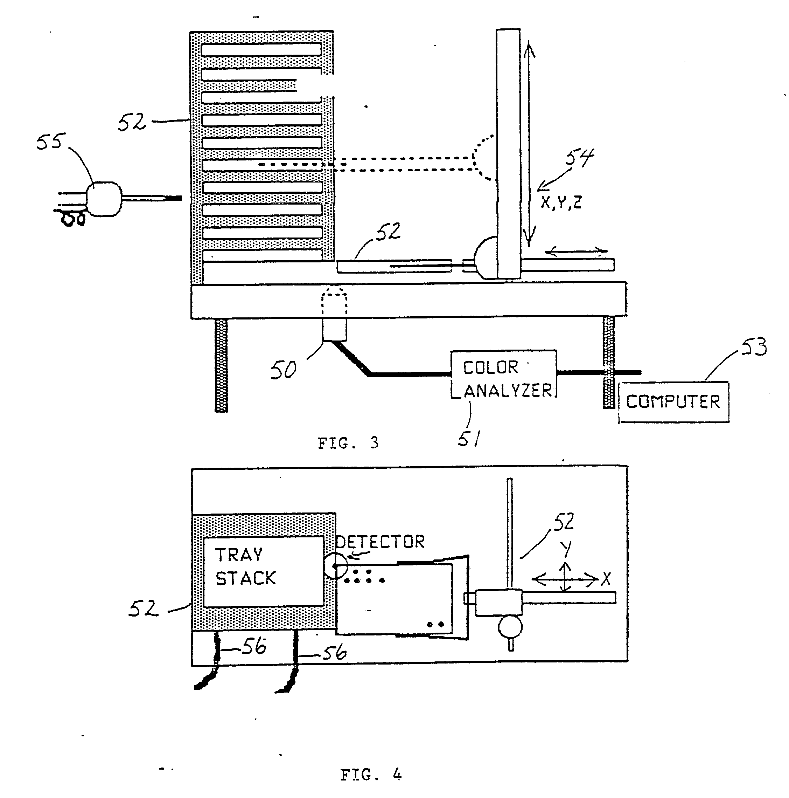

Figure 3 illustrates one method by which the microcolorimeter of the present invention can be incorporated into an overall laboratory system. A microcolorimeter 50 and a color analyzer 51 are illustrated in Figure 3. Illustrated above the microcolorimeter 50 is a plate of microtiter wells 52. Also illustrated in Figure 3 is a computer 53 attached to the color analyzer.

It will be appreciated that the plate of microtiter wells 52 may be moved above the microcolorimeter 50 such that all of the samples contained within the microtiter well plate 52 may be read at any particular point in time.

The present invention may incorporate robotics, generally designated 54 for the purpose of moving plates of microtiter wells into position at a desired moment in time. Once the reading has been made, a new well along the same plate may be read, or in the alternative a new plate of microtiter wells may be moved into position. Robotics provides the capability of measuring samples at desired intervals even over extended periods of time. Several samples can be read at different intervals. For example, tests for several different characteristics could occur simultaneously.

Robotics systems usable in connection with the present invention include an analyzer robot to continuously position wells for readings and a sampler robot 55 to prepare samples and plates for use by the former. A Zymark robot is capable of sample preparation.

Figure 3 illustrates a stack of plates 52 containing microtiter wells. It will be appreciated that any of these plates 52 may be moved into position under the microcolorimeter 50 at any desired point in time by the robotics 54. Each microtiter plate 52 contains numerous microtiter wells, traditionally 24 to 96 depending upon sample size.

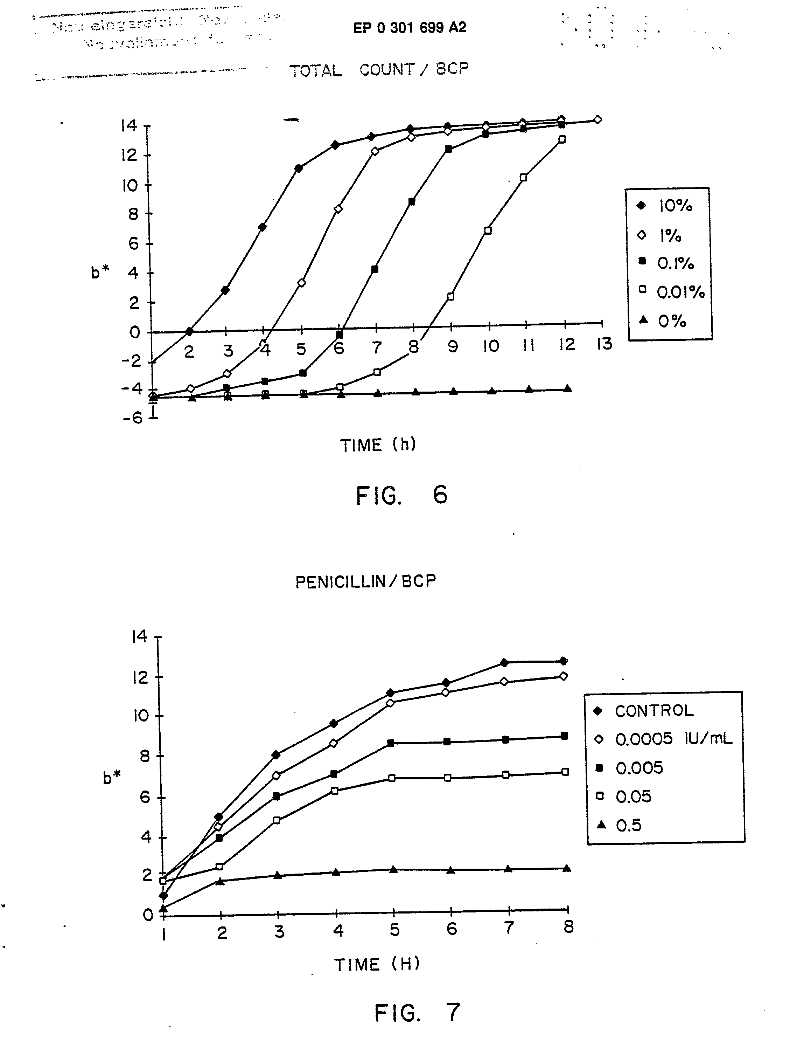

Figure 4 is a top horizontal plan view of the device as illustrated in Figure 3. Plates 52 of microtiter wells are illustrated as are the connections 56 to microcolorimeter 50. It will be appreciated from the arrows in Figure 4 that the plates of microtiter wells 52 may be moved in either the X or Y direction in order to read each of the microtiter wells contained within each plate. This will be done by the analyzer robot 52.

Thus, it will be appreciated that the present invention provides immense flexibility in reading biological samples. Many samples can be read within a very short period of time. At the same time, the samples can be reread at any desired interval. Thus, accurate data concerning biological changes within the system can be provided even overnight without the need for continuous human monitoring.

From the discussion above, the method and manner of use of the present invention can be readily appreciated. A sample will be prepared as appropriate. Using the present invention this may simply entail placing the sample in a clean container such as a microtiter well. The sample container is then placed adjacent the optical opening of the microcolorimeter apparatus.

Once the sample is properly prepared and in place, the microcolorimeter is placed into operation. An energy source will produce a burst of light which will strike the surface of the sample. The light beam reflected by the sample is then directed into a series of L*, a*, and b* detectors. These detectors detect at least one of the tristimulus color values. The process of directing light into the sample and detecting the reflected light is repeated as necessary. It will be appreciated that the entire process may take a period of days.

The data obtained is analyzed by associated computer software and hardware and may be plotted. From the plotting of data over time characteristics such as bacteria count of the sample can easily be determined.

### 3. Experimental Results

Various runs have been made which illustrate the utility of the present invention. The measurements include those for various different characteristics of biological fluids.

Figure 5 summarizes typical readings of the L*, a*, b* tristimulus readings associated with a colorimeter. These data were obtained by reading the reflected color on the bottom of microtiter plate wells. The wells were filled with samples comprising water and water with added brom cresol purple (BCP), and adjusted to pH 4. Other wells were filled with the same materials at pH 7 (more blue or a low b* readings).

As shown further in Figure 5, wells filled with milk had higher L* readings at pH 7 than at pH 4. Differences in a* and b* are also evident. This indicates that any of the three parameters will be usable in the measurement of pH changes in the medium, even when only one dichromatic dye is used. The data also show that the system is usable in clear solutions. A readable reflectance measurement can be made indicating that turbidity and absorbance measurements do not require separate instrumentation.

Figure 6 illustrates the change in b* when lactic culture was inoculated in sterile milk in the microtiter wells. As the log dilutions of culture from 10% down to .01% were measured, there were longer equal times associated with the developing slopes. This is as expected and what one would find in an impedance microbiology system. These data demonstrate the ability of the instrument to quantitate the microflora in a substance and to predict shelf life of a food or similar type of product. The data further clearly indicate that the present invention is capable of duplicating data now obtained by the impedance methods.

Figure 7 illustrates the changes in b* associated with levels of penicillin in sterile milk dyed with BCP. Sterile milk containing different levels of antibiotics were placed in wells. A 10% inoculum of lactic culture (UC310) was added to each well and the plates were incubated at 30° C.

The sensitivity of this method appears superior to reference methods and this figure indicates the ability to separate between 0.0005 and 0.0000 (control) levels, a capability not provided by existing methods. The rising lines indicate that the solution is gradually turning more yellow (i.e., high b* corresponds to more yellow and less blue).

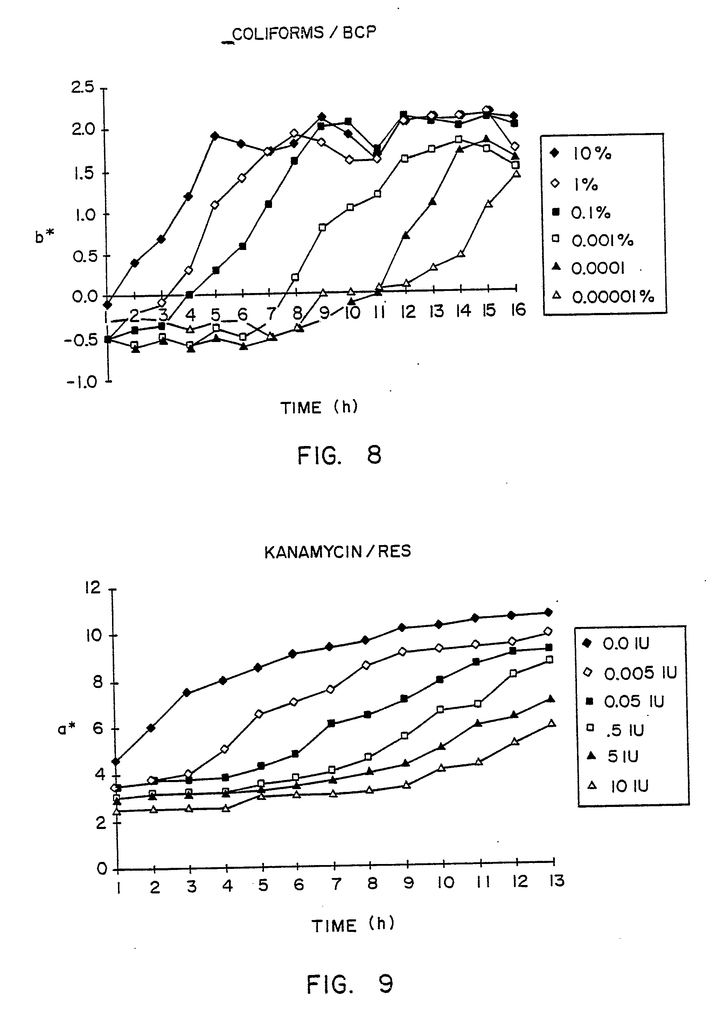

Figure 8 illustrates the changes in b* due to coliform activity. These changes are very subtle compared to those in the other figures. The range is only from -0.5 to 2.2 compared to -5 to 14 in the other figures. As illustrated in Figure 8, there is much noise around the "stable" readings. Nevertheless, it was possible using the present invention to measure the different times associated with the color changes through the slopes of the curves. This test provides the capability of counting coliforms in foods. These subtle changes are typical of those found in highly colored foods. This makes it possible to measure coliforms in chocolate ice cream, for example. The ability to predict shelf life of dark colored foods would also be provided.

Figure 9 illustrates changes in a* in an activity test like that described with reference to Figure 7, except Kanamycin antibiotic and resazurin oxidation/reduction ("O/R") dye was used. These changes can be made to occur much faster if the wells are sealed from the atmosphere using mineral oil or tape to prevent oxygen from dissolving and raising the O/R potential.

Figure 9 shows that O/R dyes can be resurrected for use in determining bioactivity. Because resazurin changes from lavender to pink to colorless, this is another dye that could use L*, a*, b* parameters separately or in combination to provide precise measurement of changes in bioactivity.

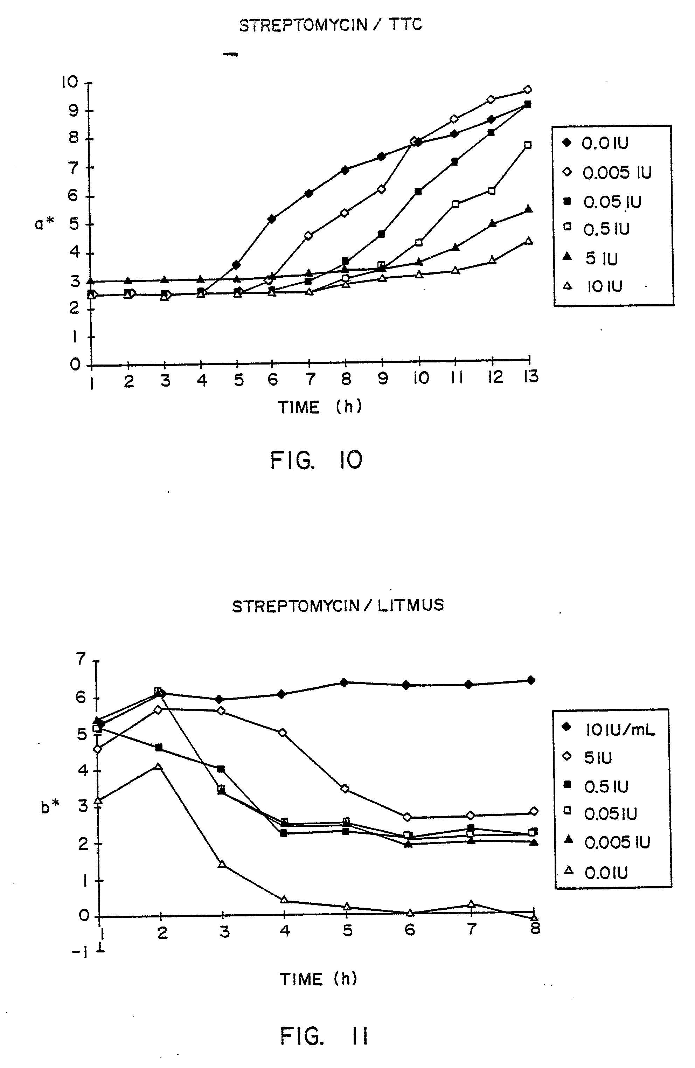

Figure 10 illustrates changes in a* in an activity test like that described with reference to Figure 7, except streptomycin antibiotic was used and triphenyl tetrazolium chloride (TTC) pH dye was used. This dye changes from colorless to red as the pH drops from 6 to 4. The a* or L* could be used separately or in combination to provide a pH endpoint in this set of measurements.

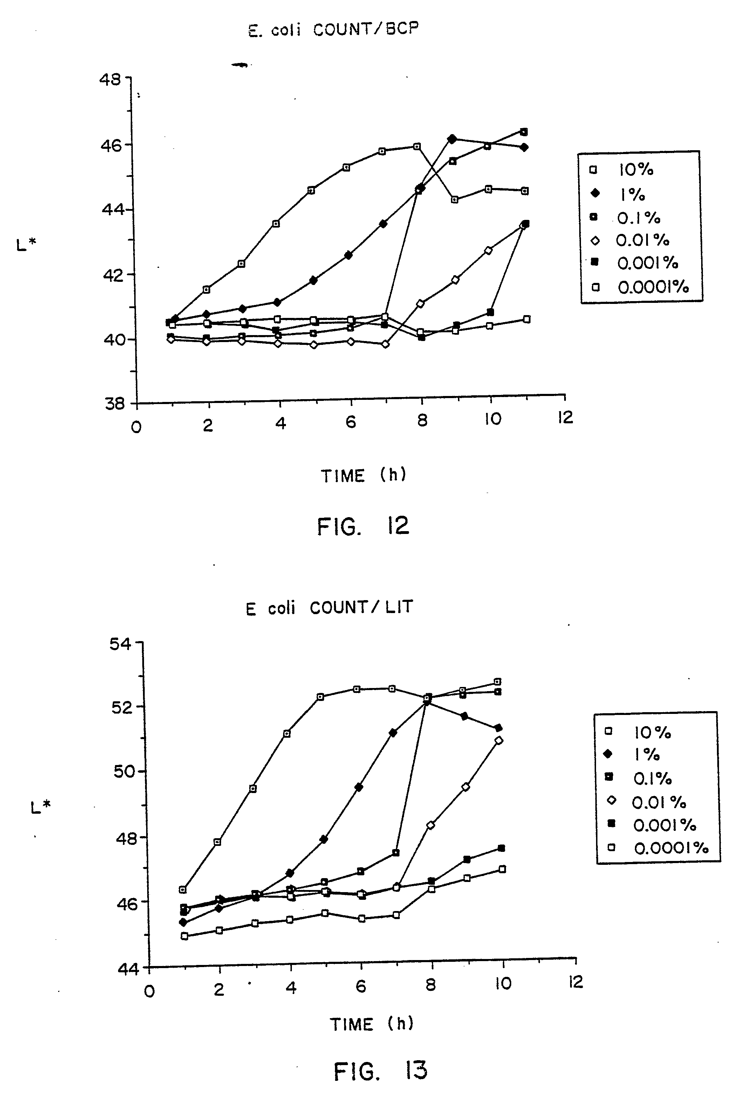

Figure 11 illustrates changes in b* when litmus dye was used in combination with streptonycin. Data are plotted to demonstrate that a classical O/R-pH indicator dye can be applied again in modern instrumentation. Changes in a* and L* could also be used to provide data in this setting.

In Figure 12, E Coli was diluted out and allowed to grow in milk containing BCP dye. The solution went from blue to yellow as was discussed above. The solution also goes from "gray" to "light gray" a shown on this Figure which contains a plot of the L* valve. The time of changing depends upon the concentration of cells present even though the L* valve changed only approximately 6 units in the experiments set forth in Figure 12. Thus, correlation of detection times and cell numbers is possible and, thus, establishes the use of all three parameters in detection modes.

Figure 13 also shows the changes of L* as litmus changes from darker to lighter shades in a solution containing E Coli. This data also clearly points out that the present invention is effective for detecting bacteria concentration in a biological mixture.

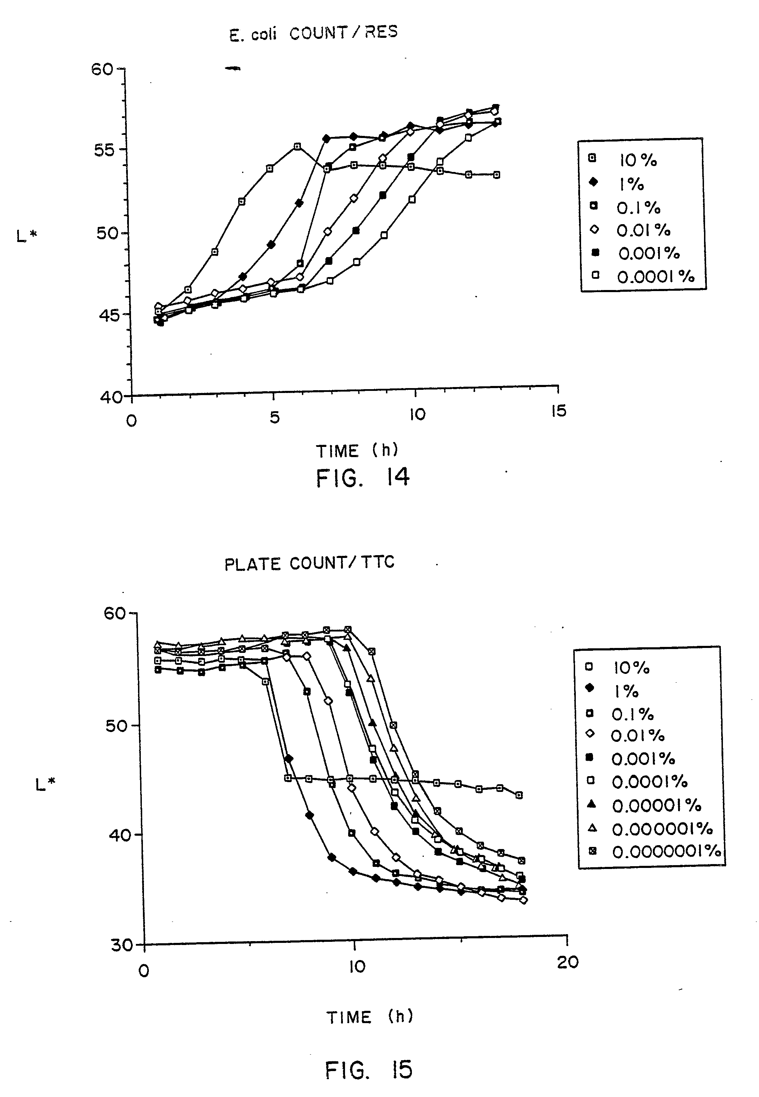

Figure 14 shows data obtained using resazurin dye in a liquid E. coli. Again, very fine changes were detected. Resazurin dyes classically go from colored to colorless and these experiments show that this is measurable across a range of 10 L* units.

Figure 15 illustrates plate count data obtaining using TTC dye. As shown in the figure, the TTC dye starts out colorless (i.e., high L* valve) and develops a red color after acid is produced in the medium. In this Figure, the numbers of bacteria can be estimated as the media changes from colorless to pink across a range of about 30 L* units. As can be seen with regard to the 10% sample something stopped color development but did not prevent estimation of numbers since the curve preceded any of the others in development time. Thus, plate count data can be quickly and accurately provided using the present invention.

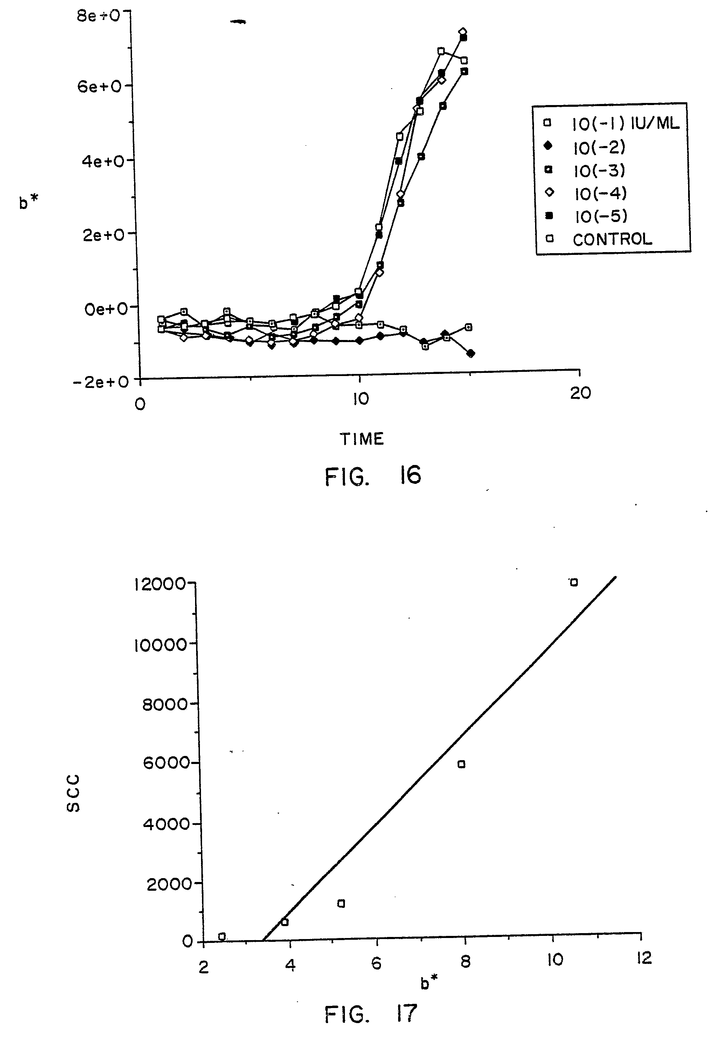

In Figure 16 the lines represent color changes in BCP used in the standard method for antibiotics detection. Spores of Bacillus sterothermophilus are suspended in agar. Milk is added on top of the agar and incubated 2.5 hours at 65° C. If the color turns yellow, no antibiotics are present. If the color remains purple, antibiotics are present above about 0.005 international units ("IU") penicillin equivalent, per milliliter. This test was modified as illustrated in Figure 16 in that instead of using petri dishes the agar was layered into Microtiter plate wells and the milk added on top. The time intervals on the Figure are 15 minutes, thus the 10 mark is actually 10 x 15 = 150 minutes or 2.5 hours.

The color change to yellow occurred rapidly with all samples containing levels of antibiotics of 0.001 and below. The samples with 0.01 and 0.1 IU/mL equivalents remained purple and the b* reading was distinct.

In Figure 17 the lines represent color changes during incubation of the Delvotest Multi for antibiotics. This approach uses the same test culture and conditions described in Figure 16. A prefilled tray is provided so that no local preparation is necessary. The instrument provides automated readout of the results. The time intervals are 15 minutes, thus the large color changes occurred in 6 x 15 = 1.5 hours. When comparing the results of Figures 16 and 17 with tat of Figures 7, 9, and 10 it is evident that the embodied methodologies provide better quantitative estimations of antibiotics than the present reference methods.

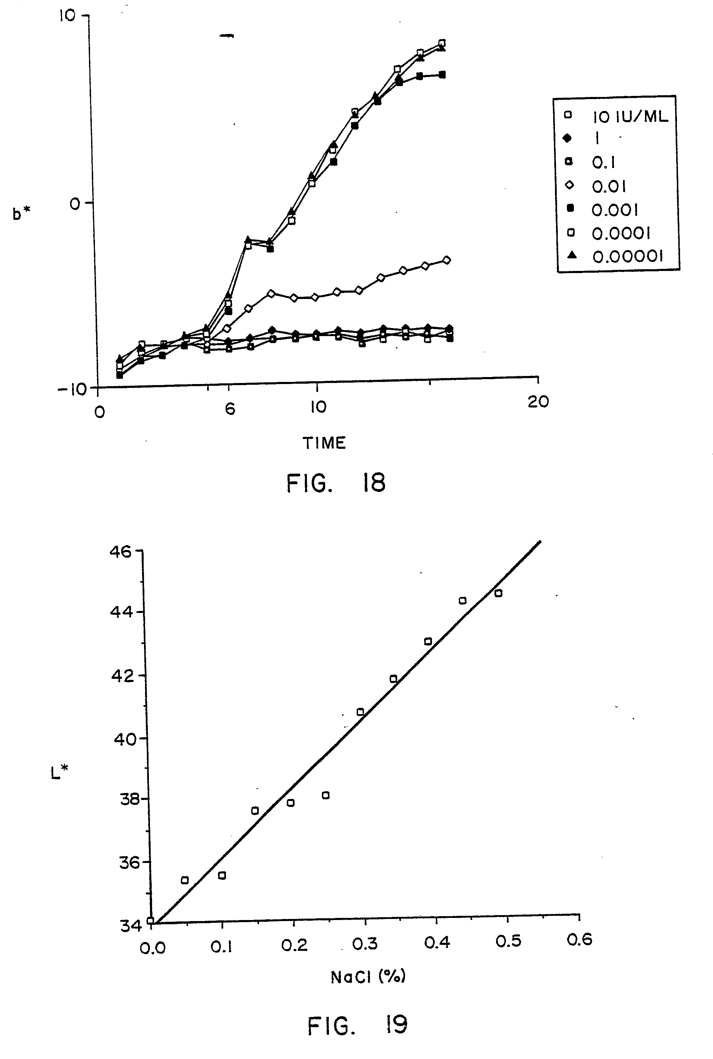

Figure 18 is a linear plot comparing the somatic cell count (SCC) in milk with a colorimetric test for chloride ion in milk, as plotted with the $b^*$ value. Known samples of abnormal milk were obtained from the Utah State University Dairy Center. The Mohr test for chloride ion was used. Sodium hydroxide and potassium dichromate were added to microtiter plate wells. Milk was added followed by a solution of silver chloride.

The yellow color reaction was immediate, stable, and did not require boiling. It should be noted that the range for the $b^*$ values from 100 to 1 million SCC was wider than was the SCC range on the y axis. Thus it appears that measurement of chloride ion would help predict mastitic milk in addition to pH differences.

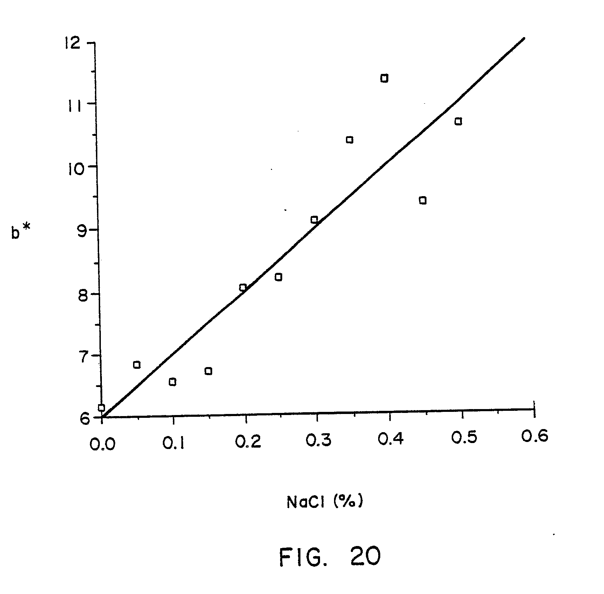

In Figures 19 and 20 milk had increasing levels of NaCl added to develop a standard curve (as described by Okigbo et al., "Portable Conductivity Meter for Detecting Abnormal Milk", J. Dairy Sci. 67:1510, 1984) usable for measurement of abnormal milk. See also, Khayat and Richardson, "Detection of Abnormal Milk with Impedance Microbiology Instrumentation," J. Food Protection 49:519 (1986). One hundred microliters of milk, 10 uL distilled water, 50 uL 1 % NaOH, and 50 uL 5% $K_2Cr_2O_7$, were mixed in wells. Then 125 uL 0.1 M $AgNO_3$ were mixed in and the color measured immediately. The results are plotted in these two figures. The precision appears superior to using pH measurements for abnormal milk. These figures demonstrate the superiority of using the $L^*$ value plotted in Figure 9 (R = 0.99) to the $b^*$ value plotted in Figure 20 (R = 0.92). Thus, detector mode changes will be dictated by the specific parameters of the assay.

## Summary

In summary, the present invention accomplishes all of the objects set forth above. The present invention provides methods for determining bioactivity in a biological mixture which overcome many of the serious problems encountered in the prior art. For example, it is not necessary using the present invention to engage in extensive preincubation, culturing, and detector stabilization of the mixture to be measured. Likewise, using the present invention numerous samples can be run rapidly and results can be obtained in one hour or less for certain measurements. This period of time for obtaining results is unheard of in the trade for a versatile instrument.

The present invention also provides the ability to measure various different characteristics using the same apparatus. Indeed, some of the different characteristics may be measured simultaneously by measuring changes in the $L^*$, $a^*$, or $b^*$ values or reflected visual spectrum light.

In addition, it is not necessary to measure extremely large volumes of sample as has been the case in the past. Furthermore, extremely low concentrations of bacteria and other types of microorganisms can be detected using the present invention.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A method for determining bioactivity of a biological sample characterised in that it comprises the steps of:

(a) directing a pulse of visual spectrum light into the biological sample such that a portion of the light is reflected out of the sample;

(b) detecting the light reflected from the sample;

(c) separating the color components of the light reflected from the sample; and

(d) using measuring means to measure the color characteristics of at least one of the color components of the reflected light over a period of time in order to determine changes in the bioactivity of the biological sample.

2. A method according to claim 1 characterised in that tristimulus visual spectrum light is employed and at least one of the $L^*$, $a^*$ and $b^*$ values are measured.

3. A method for determining bioactivity characterised in that it comprises the steps of:

(a) obtaining a biological sample;

(b) directing a pulse of visual spectrum light into the sample;

(c) providing measuring means in the form of a microcolorimeter for measuring the reflectance of the pulse of light after it has passed into the sample;

(d) measuring at least one of the L*, a*, and b* values of the reflected light in order to determine the bioactivity of the sample.

4. A method according to claim 2 or claim 3 characterised in that at least two of the L*, a* and b* values are measured.

5. A method according to claim 4 characterised in that all three L*, a* and b* values are measured.

6. A method according to any of the preceding claims characterised in that the biological sample is substantially opaque and that light reflected from the surfaces of the opaque sample is measured.

7. A method according to any of the preceding claims characterised in that it comprises determining the bioactivity of milk.

8. A method according to any of claims 1 to 5 characterised in that the sample is substantially clear.

9. A method according to claim 8 characterised in that a mirrored surface is placed on at least one side of the biological sample such that a portion of the pulse of light is reflected by the mirror into the measuring means.

10. A method according to any of the preceding claims characterised in that the biological sample contains a dye which allows the pH of the sample to be measured by measuring the change in color of the dye.

11. A method according to any of claims 1 to 9 characterised in that the biological sample contains a dye which allows the oxidation/reduction characteristics of the sample to be measured.

12. A method according to any of the preceding claims characterised in that total bacteria count, pH or sterility is determined by observing changes in the color characteristics of the reflected light.

13. A method according to claim 12 characterised in that coliform count is determined by observing changes in the color characteristics of the reflected light.

14. Apparatus for determining bioactivity in a sample using reflected visual spectrum energy characterised in that it comprises:
a container for holding a sample;
means for producing pulses of visual spectrum light;
means for directing the pulses of visual spectrum light into the sample; and
detection means for detecting the reflectance of the visual spectrum light after it has entered the sample and has been reflected by the sample.

15. Apparatus according to claim 14 characterised in that the container for holding a sample comprises a microtiter well.

16. Apparatus according to claim 14 or claim 15 characterised in that the detection means comprises a microcolorimeter.

17. Apparatus according to any of claims 14 to 16 characterised in that the means for producing pulses of visual light produces tristimulus light having measurable L*, a* and b* components at least one of which is detected by a detection means.

18. A system for determining bioactivity in a biological mixture comprising:
at least one holder for holding a sample of biological mixture;
means for producing tristimulus visual spectrum light;
means for directing the pulse of light into the sample; and
detection means for detecting the reflectance of the visual spectrum light once it has entered the sample.

19. A system for determining bioactivity in a biological mixture according to claim 18 characterised in that the holder comprises a microtiter well contained within a microtiter plate comprising a plurality of microtiter wells.

20. A system according to claim 18 or claim 19 characterised in that the means for positioning the sample in the path of the pulse of light comprises laboratory robotics.

21. A system according to any of claims 18 to 20 characterised in that detection means comprises a microcolorimeter.

22. A system according to claim 21 characterised in that it further comprises a computer in communication with the microcolorimeter capable of analyzing the relected light detected by the colorimeter such that bioactivity can be determined.

14

12

20

16    A    B    A    16

18    O    O

18    C

10

22

R    B    G    24

**FIG. 1**

34

R,G,B, DETECTORS

38

DETECTOR MODULE

FIBER OPTICS

36

MICROTITER WELL

REFRACTED
REFLECTED
ENERGY
BEAMS

40

OPAQUE SAMPLE

28

32

ENERGY, X,Y
POSITIONING
MODULE

R,G,B, REFERENCE
DETECTORS

26

ENERGY SOURCES

LINEAR ENERGY BLOCKING RING    30

**FIG. 2**

FIG. 3

FIG. 4

TOTAL COUNT / BCP

FIG. 6

PENICILLIN / BCP

FIG. 7

COLIFORMS / BCP

FIG. 8

KANAMYCIN / RES

FIG. 9

STREPTOMYCIN / TTC

FIG. 10

STREPTOMYCIN / LITMUS

FIG. 11

EP 0 301 699 A2

E. coli COUNT/BCP

Legend:
- □ 10%
- ◆ 1%
- ▣ 0.1%
- ◇ 0.01%
- ■ 0.001%
- ▫ 0.0001%

FIG. 12

E coli COUNT/LIT

Legend:
- □ 10%
- ◆ 1%
- ▣ 0.1%
- ◇ 0.01%
- ■ 0.001%
- ▫ 0.0001%

FIG. 13

E. coli COUNT / RES

FIG. 14

PLATE COUNT / TTC

FIG. 15

FIG. 16

FIG. 17

FIG. 18

FIG. 19

NaCl (%)

FIG. 20